# EUROPÄISCHE PATENTSCHRIFT

(11) **EP 3 773 083 B1**
(45) Veröffentlichungstag und Bekanntmachung des Hinweises auf die Patenterteilung: **26.02.2025**
(21) Anmeldenummer: 19723634.2
(22) Anmeldetag: 11.04.2019
(51) Int. Cl.: A47C 27/20, A61G 7/02

(54) **VORRICHTUNG ZUM ABLEITEN VON URIN**
DEVICE FOR DISCHARGING URINE
DISPOSITIF D'ÉVACUATION DE L'URINE

(30) Priorität: 12.04.2018 CH 4662018
(43) Veröffentlichungstag der Anmeldung: 17.02.2021
(73) Patentinhaber: Isik, Hasan Hüseyin, 9500 Wil SG 1 (CH)
(72) Erfinder: Isik, Hasan Hüseyin, 9500 Wil SG 1 (CH)
(74) Vertreter: Frommhold, Joachim
(86) Internationale Anmeldenummer: PCT/CH2019/000012
(87) Internationale Veröffentlichungsnummer: WO 2019/195950

(56) Entgegenhaltungen:
- US-A- 183 973
- US-A- 2 654 898
- US-A- 3 345 652

## Beschreibung

Die Erfindung betrifft eine Vorrichtung zum Ableiten von Urin, insbesondere zum verunreinigungsfreien zum Ableiten von Urin von einer, auf einer Liegestätte oder Sitzfläche befindlichen Person,

Spritzverunreinigungen können beim sogenannten Stehurinieren, insbesondere von Männern praktiziert, auftreten. Es wurden daher bereits verschiedene Urinierhilfen, auch für Frauen, vorgeschlagen, die ein spritzfreies zum Ableiten von Urin in ein WC ermöglichen sollen, zum Beispiel schlauch- oder röhrenförmige Urinierhilfen gemäss DE 19916283 A1 oder DE 10103910 B4.

Darüberhinaus kann auch bei eingeschränkter Mobilität der betroffenen Personen ein Urinieren odereine Defäkation nur bedingt und/oder nicht verunreinigungsfrei gegeben sein. So bei bettlägerigenPersonen in Spitälern oder Pflegeheimen, die auf Hilfsmittel wie Schieber oder Bettpfannen und dieUnterstützung von Dritten angewiesen sind. Dies ist für die betroffenen Personen unangenehm undes kann ohne Unterstützung Dritter zu Verunreinigungen des Bettes kommen und verursacht zusätzlichen Reinigungs- und Pflegeaufwand.

Die Aufgabe der Erfindung ist es daher, eine Vorrichtung zum Ableiten von Urin zu schaffen, die mit geringem Aufwand ein hygienisches Urinieren im Liegen oder Sitzen ermöglicht.

Die Aufgabe ist mit den Merkmalen des Patentanspruchs 1 gelöst.

Erfindungsgemäss ist eine Liegestätte in Form einer Matratze eines Betts mit einem Einschnitt versehen, der sich durchgehend über die Dicke der Matratze erstreckt. Im Bereich des Einschnitts ist ein Einsatz angeordnet. Der Einschnitt ist manuell oder mittels einer Aufspannanordnung aufweitbar, so dass der Einsatz mit geringem Kraftaufwand entsprechend entnehmbar und einsetzbar ist.

Für eine sehr kraftarme Handhabung ist erfindungsgemäss auf oder unter dem Einsatz im Einschnitt eine Aufspannanordnung angebracht.

Weitere vorteilhafte Ausgestaltungen der erfindungsgemässen Vorrichtung sind in den abhängigen Patentansprüchen offenbart.

So ist die Aufspannanordnung erfindungsgemäss in Form eines Aufspannrahmens ausgebildet.

Die erfindungsgemässe Vorrichtung ermöglicht neben einem verschmutzungsfreien Ableiten des Urins auch eine hygienische und geruchsneutrale Lagerung desselben bis zur Entsorgung.

Die erfindungsgemässe Vorrichtung ist insbesondere zum Urinieren von Männern geeignet, kann aber auch an die körperlichen Gegebenheiten von Frauen angepasst werden.

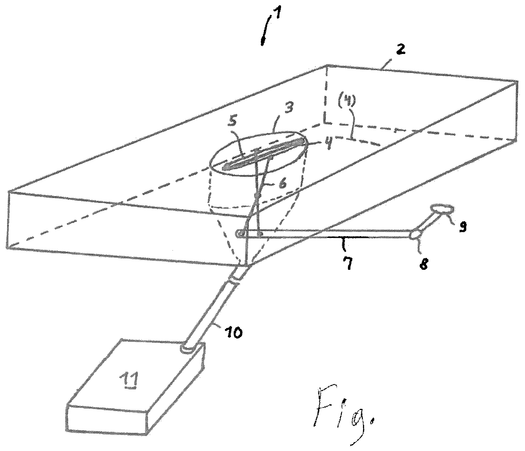

Die Erfindung wird nachfolgend in einem Ausführungsbeispiel anhand einer Zeichnung näher beschrieben. Die Zeichnung zeigt die erfindungsgemässe Vorrichtung am Beispiel eines Bettes in einer Figur.

Ein normales Bett 1 ist mit einer Matratze 2 ausgestattet, die im Bereich des Unterbauchs mit einem Einschnitt 4 versehen ist, der elastisch öffnenbar und verschliessbar ist und der sich durchgehend über die Dicke der Matratze 2, parallel zu den Längsseiten der Matratze 2 verlaufend, erstreckt und der etwa gleichweit von den Längsseiten der Matratze 2 beabstandet ist. Der Einschnitt 4 weist bevorzugt eine Länge von mindestens 20cm auf und kann auf wenigstens 5cm geöffnet/geweitet werden. Der Einschnitt 4 ist in der Figur vereinfacht, in geschlossenem Zustand, mit aneinander liegenden Seitenwänden dargestellt.

Die Matratze 2 ist mit einem Laken überzogen, das im Bereich des Einschnitts 4 ebenfalls eingeschnitten ist und im Umfeld des Einschnitts 4 mit einem Einsatz 3 aus hautfreundlichen, wasserabweisendem und leicht reinigbarem Material versehen ist. Der Einsatz 3 erstreckt sich auch entlang der Seitenwände des Einschnitts 4 bis zur Unterseite der Matratze 2. Der Einsatz 3 kann ein Teil des Lakens sein oder als separates Element auf dieses aufgebracht resp. in den Einschnitt 4 eingebracht sein. Zudem kann die Matratze 2 in Richtung des Einschnitts 4 eine Senke aufweisen.

Alternativ oder ergänzend kann die Matratze 2 auch mit einem Einschnitt versehen sein, der näher an einer Längsseite der Matratze 2 angeordnet ist und der etwa parallel zu den Schmalseiten der Matratze 2 verläuft, wie in der Figur angedeutet.

Im Einschnitt 4 ist auf oder unter dem Einsatz 3 ein Aufspannrahmen 5 angeordnet, der mit einem Scherenhebel 6 ausserhalb der Matratzenunterseite verbunden ist.

Der Aufspannrahmen 5 und der Scherenhebel 6 bestehen bevorzugt aus einem korrosionsfesten Material, zum Beispiel einem Leichtmetall oder aus einem belastbaren Kunststoff.

Der Aufspannrahmen 5 kann auch zangenförmig oder anderweitig ausgebildet sein. Er muss unabhängig von der Bauform bei Betätigung des Scherenhebels 6 in der Lage sein, den Einschnitt zu weiten.

Ebenso In einer nicht-erfindungsgemässen Ausführung kann anstelle des Scherenhebels 6 ein Zahnsegment verwendet werden oder im Einschnitt 4 eine Faltmembran angeordnet sein, die auf Druck öffnet.

Die dem Aufspannrahmen 5 gegenüberliegenden Hebelenden des Scherenhebels 6 sind mit einem Hebel 7 (Zugstange, Zugseil o. a.) gekoppelt, dessen anderes Ende mit einem Gelenk 8 versehen ist, das mit einem Handgriff 9 in Wirkverbindung befindlich ist. Dadurch wird eine Öffnungs- und Schliessbewegung des Scherenhebels 6 resp. des Aufspannrahmens 5 ermöglicht (Fig.). Während einer Öffnungsbewegung des Aufspannrahmens 5 wird das Material der Matratze im Einschnitt 4 verdrängt und ein offener Bereich geformt.

Weiterhin kann der Scherenhebel 6, ggf. eingeschlossen Hebel 7, auch motorisch betätigt werden, wobei insbesondere Elektroantriebe oder pneumatische Antriebe zur Anwendung gelangen. Bevorzugt kann der Scherenhebel 6 mittels Links-Rechts-Umschaltbewegung geöffnet oder geschlossen werden. Der Antrieb kann zum Beispiel mittels eines drahtgebundenen Tasters drahtlos über eine Gestensteuerung oder mittels App betätigt werden.

Anstelle des Aufspannrahmens kann die Aufspannanordnung auch in Form einseitig gelenkig verbunden angeordneter Platten ausgebildet sein. Sie sind im geschlossenen Zustand aneinander anliegend und können zum Beispiel mittels einer drehbaren Stange V-förmig geöffnet werden.

In anderer Ausgestaltung können die zwei Platten auch mittels Zylinder oder Schnecke relativ zueinander beweglich angeordnet sein, ohne gelenkige Verbindung miteinander.

Es ist in vereinfachter Ausgestaltung ebenfalls möglich, auf Aufspannelemente zu verzichten und den Einschnitt 4 manuell zu weiten und entsprechend auch den Einsatz 3 manuell zu hanhaben.

Der Einschnitt 4 geht an der Unterseite oder unterhalb der Matratze 2 zugleich flüssigkeits- und geruchsdicht in eine Leitung 10, zum Beispiel ein Schlauch, zu einem Tank 11 zur Sammlung von Urin und ggf. von Fäkalien über. Die Leitung 10 ist ausreichend schräg verlegt, um vollständig entleert zu werden.

Anstelle des Schlitzes könnte auch lediglich eine feste Aussparung (rund oder oval) mit Einsatzteil (manuell oder motorisch betrieben) vorgesehen sein.

Zur Benutzung der erfindungsgemässen Vorrichtung muss die im Bett 1 liegende Person den Handgriff 9 so betätigen, dass mittels des Scherenhebels 6 der Einschnitt 4 soweit geöffnet wird, dass zum Beispiel ein Penis darüber oder darin platzierbar ist, so dass die Person ihr Wasser ausschliesslich und spritzfrei in den Einschnitt 4 lösen kann.

Mit der Betätigung des Handgriffs 9 wird, in bevorzugter Ausführung, zugleich eine Klappe (nicht dargestellt) an der Verbindung der Leitung 10 mit dem Tank 11 geöffnet, so dass aller Urin in den Tank 11 gelangt und keine Flüssigkeit in der Leitung verbleibt. Manuell oder nach voreingestellter Zeit wird nach Abschluss des Vorgangs die Klappe wieder geschlossen.

An sich fungiert bereits der geschlossene Einschnitt 4 als Geruchsverschluss.

Zur Desinfektion/Trocknung der erfindungsgemässen Vorrichtung, insbesondere der Aufspannanordnung kann UV-Licht und/oder ein Desinfektionsspray in den Schlitz oder das Einsatzteil eingebracht werden. Dies kann manuell erfolgen oder mittels eines Sensor, auf die Offenstellung und/oder Feuchte reagiert.

Die Person oder eine Drittperson kann den Tank 11 in notwendigen zeitlichen Abständen leeren und reinigen.

Die erfindungsgemässe Vorrichtung kann auch in einer Sitzfläche oder im Bereich der Vorderkante eines Stuhls oder Fahrersitzes, an den Sitz angepasst, angeordnet sein. Somit ist ein Urinieren auch im Sitzen oder für Kraftfahrer während der Fahrt resp. im Stau möglich.

### Aufstellung der Bezugszeichen

1 Bett
2 Matratze
3 Einsatz
4 Einschnitt
5 Aufspannrahmen
6 Scherenhebel
7 Hebel
8 Gelenk
9 Handgriff
10 Leitung
11 Tank

## Patentansprüche

1. Vorrichtung zum Ableiten von Urin, insbesondere zum verunreinigungsfreien Ableiten von Urin, von auf einer Liegestätte oder Sitzfläche befindlichen Person, wobei die Vorrichtung eine Liegefläche aufweist, die eine Matratze (2) ist, die mit einem Einschnitt (4) versehen ist, der sich durchgehend über die Dicke der Matratze (2) erstreckt, und der öffnen- und schliessbar ist, wobei im Bereich des Einschnitts (4) ein Einsatz (3) angeordnet ist und wobei auf oder unter dem Einsatz (3) im Einschnitt (4) eine Aufspannanordnung in Form eines Aufspannrahmens (5) angeordnet ist, **dadurch gekennzeichnet, dass** der Aufspannrahmen (5) mit einem Scherenhebel (6) in Wirkverbindung steht.

2. Vorrichtung nach Anspruch 1, **dadurch gekennzeichnet, dass** der Scherenhebel (6) mit einem Hebel (7) gekoppelt ist, dessen anderes Ende mit einem Gelenk (8) versehen ist, das mit einem Handgriff (9) in Wirkverbindung befindlich ist.

3. Vorrichtung nach Anspruch 1 oder 2, **dadurch gekennzeichnet, dass** die Aufspannelemente relativ zueinander bewegliche Platten sind.

4. Vorrichtung nach einem der Ansprüche 1 bis 3, **dadurch gekennzeichnet, dass** der Einschnitt (4) elastisch öffnenbar und verschliessbar ist.

5. Vorrichtung nach einem der Ansprüche 1 bis 4, **dadurch gekennzeichnet, dass** der Einschnitt (4) an der Unterseite oder unterhalb der Matratze (2) in eine Leitung (10) zu einem Tank (11) übergeht.

6. Vorrichtung nach einem der Ansprüche 1 bis 5, **dadurch gekennzeichnet, dass** der Einschnitt (4) etwa parallel zu den Längsseiten und/oder Schmalseiten der Matratze (2) verlaufend ausgebildet ist.

7. Vorrichtung nach Anspruch 1, **dadurch gekennzeichnet, dass** der Scherenhebel (6) mit einem Antrieb, bevorzugt einem Elektroantrieb oder einem pneumatischen Antrieb gekoppelt ist.

## Claims

1. Device for draining urine, in particular for the contamination-free draining of urine from a person located on a bed or seat, wherein device comprises a lying surface, which is a mattress (2) that is provided with a cutout (4) that extends continuously through the thickness of the mattress (2) and that can be opened and closed, wherein an insert (3) is arranged in the region of the cutout (4) and wherein a clamping arrangement in the form of a clamping frame (5) is arranged on or under the insert (3) in the cutout (4), **characterized in that** the clamping frame (5) is operatively connected to a scissor lever (6).

2. Device according to claim 1, **characterized in that** the scissor lever (6) is coupled to a lever (7) whose other end is provided with a joint (8) that is operatively connected to a handle (9).

3. Device according to claim 1 or 2, **characterized in that** the clamping elements are plates that can be moved relative to one another.

4. Device according to one of the claims 1 to 3, **characterized in that** the cutout (4) can be opened and closed elastically.

5. Device according to one of the claims 1 to 4, **characterized in that** the cutout (4) on the underside or underneath the mattress (2) merges into a line (10) leading to a tank (11).

6. Device according to one of the claims 1 to 5, **characterized in that** the cutout (4) is designed to run approximately parallel to the longitudinal sides and/or narrow sides of the mattress (2).

7. Device according to claim 1, **characterized in that** the scissor lever (6) is coupled to a drive, preferably an electric drive or a pneumatic drive.

## Revendications

1. Dispositif pour l'évacuation de l'urine, en particulier pour l'évacuation de l'urine sans contamination, d'une personne se trouvant sur une couchette ou sur une assise, dans lequel le dispositif présente une surface de couchage qui est un matelas (2) qui est pourvu d'une entaille (4) qui s'étend en continu sur l'épaisseur du matelas (2) et qui peut être ouverte et fermée, dans lequel un insert (3) est disposé dans la zone de l'entaille (4) et dans lequel un système de serrage sous la forme d'un cadre de serrage (5) est disposé sur ou sous l'insert (3) dans l'entaille (4), **caractérisé en ce que** le cadre de serrage (5) est en liaison fonctionnelle avec un levier en ciseaux (6).

2. Dispositif selon la revendication 1, **caractérisé en ce que** le levier en ciseaux (6) est accouplé à un levier (7) dont l'autre extrémité est pourvue d'une articulation (8) qui se trouve en liaison fonctionnelle avec une poignée (9).

3. Dispositif selon la revendication 1 ou 2, **caractérisé en ce que** les éléments de serrage sont des plaques mobiles les unes par rapport aux autres.

4. Dispositif selon l'une des revendications 1 à 3, **caractérisé en ce que** l'entaille (4) peut être ouverte et fermée de manière élastique.

5. Dispositif selon l'une des revendications 1 à 4, **caractérisé en ce que** l'entaille (4) se prolonge, au niveau de la face inférieure ou en dessous du matelas (2), dans une conduite (10) menant à un réservoir (11).

6. Dispositif selon l'une des revendications 1 à 5, **caractérisé en ce que** l'entaille (4) est réalisée de manière à s'étendre approximativement parallèlement aux côtés longitudinaux et/ou aux côtés étroits du matelas (2).

7. Dispositif selon la revendication 1, **caractérisé en ce que** le levier en ciseaux (6) est accouplé à un entraînement, de préférence un entraînement électrique ou un entraînement pneumatique.
